# EUROPEAN PATENT APPLICATION

(11) **EP 4 678 109 A1**
(43) Date of publication of application: **14.01.2026**
(21) Application number: 24188421.2
(22) Date of filing: 12.07.2024
(51) Int. Cl.: A61B 6/03, A61B 6/50, A61B 6/00

(54) **SYSTEM AND METHOD FOR CARDIAC CT GUIDANCE**

(71) Applicant: Koninklijke Philips N.V., 5656 AG Eindhoven (NL)
(72) Inventor: GRASS, Michael, Eindhoven (NL); BUELOW, Thomas, Eindhoven (NL); YOUNG, Stewart Matthew, 5656AG Eindhoven (NL)
(74) Representative: Philips Intellectual Property & Standards

(57) **Abstract**

A system and method for providing guidance during a cardiac CT imaging procedure. Scan data for the cardiac CT imaging procedure is received and processed to determine one or more criteria to be met during the cardiac CT imaging procedure. Video data of a room in which the cardiac CT imaging procedure is being conducted is received and processed to determine whether the one or more criteria are met. Guidance is generated based on whether the one or more criteria are met, and a user-perceptible output indicative of the guidance is provided.

## Description

### FIELD OF THE INVENTION

The invention relates to the field of cardiac CT imaging.

### BACKGROUND OF THE INVENTION

Cardiac CT imaging is used in the diagnosis of a variety of heart conditions. Cardiac CT is one of the most complex types of CT examination. A wide range of factors must be taken into account when conducting a cardiac CT scan, resulting in poor quality imaging data when cardiac CT scans are carried out by less experienced operators.

### SUMMARY OF THE INVENTION

The invention is defined by the claims.

According to examples in accordance with an aspect of the invention, there is provided a computer-implemented method for providing guidance to an operator conducting a cardiac CT imaging procedure, the computer-implemented method comprising: receiving scan data for the cardiac CT imaging procedure; processing the scan data to determine one or more criteria to be met during the cardiac CT imaging procedure, the one or more criteria comprising at least one of: one or more criteria relating to a heart rate of a subject of the cardiac CT imaging procedure; one or more criteria relating to use of an electrocardiography device; and/or one or more criteria relating to administration of a contrast agent; receiving video data of a room in which the cardiac CT imaging procedure is being conducted; processing the video data to determine whether the one or more criteria are met; generating guidance based on the determination as to whether the one or more criteria are met; and controlling a feedback unit to provide a user-perceptible output indicative of the generated guidance.

In this way, an operator may be alerted to potential problems with the CT set-up that may have a negative impact on the quality of CT imaging data, enabling the operator to correct or adjust for these issues in order to improve the quality of the CT imaging data acquired during the cardiac CT imaging procedure.

In some examples, the one or more criteria further comprises at least one criterion relating to a breathing state of the subject.

For instance, the one or more criteria may comprise a criterion defining whether a breath hold of the subject is required during the CT imaging procedure.

In some examples, the computer-implemented method further comprises processing the video data and/or CT imaging data acquired during a localizer scan of the cardiac CT imaging procedure to identify any difference between a current position of the subject and a desired position; and the generated guidance is further based on any identified difference between the current position and the desired position.

In this way, the subject may be re-positioned if re-positioning the subject would provide improved image quality.

In some examples, the computer-implemented method further comprises processing the video data and/or CT imaging data acquired during a localizer scan of the cardiac CT imaging procedure to determine an amount of metal within a scan region of the subject; and the generated guidance is further based on the determined amount of metal.

This enables an operator to remove unnecessary metal from the scan region.

In some examples, the feedback unit comprises one or more lighting devices; and the step of controlling the feedback unit to provide a user-perceptible output indicative of the generated guidance comprises controlling the one or more lighting devices to illuminate an area of the room for which an adjustment is recommended.

In this way, the operator may be provided with the generated guidance regardless of the position of the operator in the room.

In some examples, the feedback unit comprises a display device; and the step of controlling the feedback unit to provide a user-perceptible output indicative of the generated guidance comprises controlling the display device to display the video data and an overlay indicating an area of the room for which an adjustment is recommended.

In some examples, the computer-implemented method further comprises: acquiring CT imaging data for the subject; and processing the CT imaging data to determine at least one measure of image quality.

In some examples, the computer-implemented method further comprises controlling the feedback unit to provide a user-perceptible output indicative of the at least one measure of image quality.

This allows an operator to determine whether further CT imaging data of the subject is required.

In some examples, the computer-implemented method further comprises processing the video data and the at least one measure of image quality to identify any source of error.

For instance, a neural network may be trained on the video data and measure(s) of image quality to identify sources of error.

In some examples, the one or more criteria relating to the heart rate of the subject comprises one or more of: a desired heart rate range; a desired heart rate variability range; and/or a criterion that no arrythmias occur.

In some examples, the one or more criteria relating to the use of an electrocardiography device comprises one or more of: a criterion defining whether or not an electrocardiography device should be used; and/or a desired position for each of a plurality of electrodes of an electrocardiography device.

In some examples, the one or more criteria relating to administration of a contrast agent comprises one or more of: a criterion defining whether or not a contrast agent is to be administered; an amount of contrast agent to be administered; a criterion defining a contrast agent injection protocol; and/or a type of needle to be used for contrast agent administration.

There is also proposed a computer program product comprising computer program code means which, when executed on a computing device having a processing system, cause the processing system to perform all of the steps of any method described above.

According to examples in accordance with an aspect of the invention, there is provided a processing system for providing guidance to an operator conducting a cardiac CT imaging procedure, the processing system being configured to: receive scan data for the cardiac CT imaging procedure; process the scan data to determine one or more criteria to be met during the cardiac CT imaging procedure, the one or more criteria comprising at least one of: one or more criteria relating to a heart rate of a subject of the cardiac CT imaging procedure; one or more criteria relating to use of an electrocardiography device; and/or one or more criteria relating to administration of a contrast agent; receive video data of a room in which the cardiac CT imaging procedure is being conducted; process the video data to determine whether the one or more criteria are met; generate guidance based on the determination as to whether the one or more criteria are met; and control a feedback unit to provide a user-perceptible output indicative of the generated guidance.

There is also proposed a cardiac CT imaging system, comprising: the processing system described above; a CT scanner; a camera configured to acquire video data of a room in which a cardiac CT imaging procedure is being conducted; and a feedback unit configured to provide a user-perceptible output.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a cardiac CT imaging system, according to an embodiment of the invention; and
Fig. 2 illustrates a computer-implemented method for providing guidance to an operator conducting a cardiac CT imaging procedure, according to an embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system and method for providing guidance during a cardiac CT imaging procedure. Scan data for the cardiac CT imaging procedure is received and processed to determine one or more criteria to be met during the cardiac CT imaging procedure. Video data of a room in which the cardiac CT imaging procedure is being conducted is received and processed to determine whether the one or more criteria are met. Guidance is generated based on whether the one or more criteria are met, and a user-perceptible output indicative of the guidance is provided.

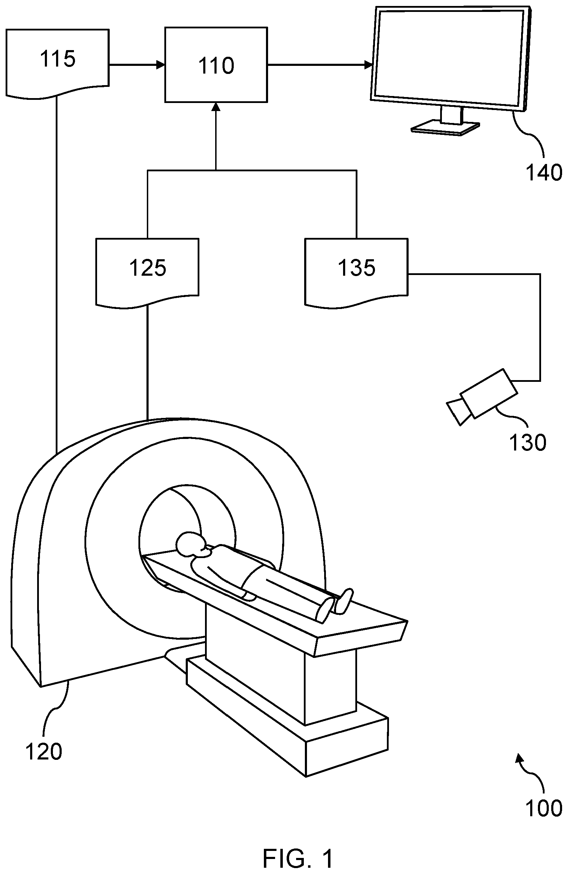

Fig. 1 illustrates a cardiac CT (computed tomography) imaging system 100, according to an embodiment of the invention. The cardiac CT imaging system comprises a processing system 110, a CT scanner 120, a camera 130 and a feedback unit 140. The processing system 110 is, itself, an embodiment of the invention.

The processing system 110 is configured to provide guidance to an operator conducting a cardiac CT imaging procedure on a subject using the CT scanner 120. The guidance may be provided prior to and/or during the cardiac CT imaging procedure (for instance, between a localizer scan, also known as a surview scan, and a main scan of the procedure, and/or during the main procedure). In some examples, the processing system 110 may be configured to provided guidance prior to and throughout the cardiac CT imaging procedure (e.g. continuously or at predefined intervals).

The processing system 110 is configured to receive scan data 115 for the cardiac CT imaging procedure. For illustrative purposes, in Fig. 1, the scan data 115 is received from the CT scanner; however, in some examples, some or all of the scan data may be received from one or more other devices, such as a memory unit.

The scan data 115 is data defining the scan to be performed during the cardiac CT imaging procedure. For instance, the scan data 115 may comprise a type of scan; for instance, the scan data may define whether the scan is to be a helical or circular scan, whether the scan is gated or triggered, and/or if the scan is a transcatheter aortic valve implantation, TAVI, CT scan, a coronary CT scan, etc. The scan data may further comprise one or more parameters of the CT scanner, for instance, the scan data may comprise one or more tube settings (e.g. one or more of: tube voltage (kV), tube current (mA) and/or focal spot size), one or more scanner settings (e.g. one or more of: rotation time, helical pitch, number of steps (in the case of a step and shoot scan), and/or whether the CT scanner is performing spectral or non-spectral imaging) and/or one or more reconstruction settings (e.g. one or more of: whether reconstruction is single-cycle or multi-cycle, target cardiac phase, slice thickness, size of field of view, position of field of view, in-plane resolution, and/or filter used). In some examples, the scan data may comprise CT imaging data acquired during a localizer scan of the cardiac CT imaging procedure, if the localizer scan has already been performed. In some examples, the scan data may comprise subject data for the subject of the cardiac CT imaging procedure (e.g. an age of the subject, a weight of the subject, whether the subject has any relevant implanted devices (such as a pacemaker, defibrillator, and/or an artificial valve), an indication of atrial fibrillation, a contrast agent incompatibility, a mean blood pressure, a mean heart rate, etc.). For instance, the scan data may comprise an electronic medical record for the subject. In some examples, the scan data may comprise one or more images and/or video data of the subject.

The processing system 110 is configured to process the scan data 115 to determine one or more criteria to be met during the cardiac CT imaging procedure. The one or more criteria comprise at least one of: one or more criteria relating to a heart rate of the subject of the cardiac CT imaging procedure; one or more criteria relating to use of an electrocardiography device; and/or one or more criteria relating to administration of a contrast agent.

For instance, where the one or more criteria comprises one or more criteria relating to a heart rate of the subject of the cardiac CT imaging procedure, the one or more criteria relating to the heart rate may comprise one or more of: a desired heart rate range, a desired heart rate variability range, and/or a criterion that no arrythmias occur. The determined one or more criteria relating to a heart rate may, for example, depend on a type of scan being performed. For instance, step and shoot scans require a stable heart rate below 70 bpm, while a helical scan without dose modulation can be carried out at higher heart rates, and can react to a varying heart rate by phase point/ECG editing post-scan. The processing system 110 may therefore, in response to the scan data 115 indicating that the type of scan is a step and shoot scan, determine criteria requiring that the desired heart rate range is below 70 bpm, with a low heart rate variability, and that no arrythmias occur. In response to the scan data indicating that the scan is a helical scan without dose modulation, the determined desired heart rate range and desired heart rate variability range may be broader, and the determined criteria may not require that no arrythmias occur.

Where the one or more criteria comprises one or more criteria relating to the use of an electrocardiography device, the one or more criteria relating to the use of an electrocardiography device may comprise a criterion defining whether or not an electrocardiography device should be used and/or a desired position for each of a plurality of electrodes of an electrocardiography device. For instance, there are some TAVI scans that are not performed in a gated fashion; for these types of scans, the processing system may determine that use of an electrocardiography device is not required. In some examples, the processing system 110 may not require the use of an electrocardiography device in response to determining that the subject has a stable heart rate, but determine a criterion specifying the use of an electrocardiography device for subjects with more unpredictable heart rates.

Where the one or more criteria comprises one or more criteria relating to administration of a contrast agent, the one or more criteria relating to administration of a contrast agent may comprise one or more of: a criterion defining whether or not a contrast agent is to be administered; an amount of contrast agent to be administered; a criterion defining a contrast agent injection protocol; and/or a type of needle to be used for contrast agent administration.

For instance, the processing system 110 may be configured to determine a criterion defining whether or not a contrast agent is to be administered based on a type of scan; for example, in response to the scan data 115 indicating that the scan is a calcium scoring scan, the processing system may determine that contrast agent administration is not required.

The processing system 110 may be configured to determine an amount of contrast agent to be administered by processing the scan data 115 to determine a measure of the size or weight of the subject of the cardiac CT imaging procedure, e.g. based on subject data for the subject in the scan data and/or based on CT imaging data acquired during a localizer scan, depending on the data available in the scan data. Additionally or alternatively, the measure of the size/weight of the subject may be determined based on an image or video data of the subject (e.g. acquired by the camera 130). The amount of contrast agent to be administered may then be determined based on the measure of the size/weight of the subject. Preferably, the measure of the size/weight of the subject is an estimate of fat-free mass of the subject (which may be derived from CT imaging data acquired during a localizer scan); the fat-free mass of a subject is particularly suitable for determining an amount of contrast agent to be administered.

In some examples, an amount of contrast agent to be administered may be included in a criterion defining a contrast agent injection protocol, which may additionally define a desired concentration of contrast agent, a flow rate, a time of contrast agent injection relative to a start time of the scan, and whether or not a saline chaser bolus is to be injected after contrast agent injection. A criterion defining a contrast agent protocol may be determined based on the measure of size or weight of the subject. If the defined contrast agent injection protocol requires a saline chaser bolus, a desired amount of saline chaser may also be specified; the desired amount of saline chaser may be also be determined based on the measure of size or weight of the subject.

In some examples, the measure of size or weight of the subject may be used to determine a type of needle to be used for contrast agent administration. The type of needle may additionally be based on a determined flow rate.

In some examples, the determined one or more criteria may include one or more further criteria. For instance, the one or more criteria may further comprise at least one criterion relating to a breathing state of the subject, such as a criterion defining whether a breath hold of the subject is required during the CT imaging procedure. The processing system 110 may be configured to determine whether a breath hold is required based on a type of scanner and/or a type of scan; for instance, a breath hold may not be required if using a 16 cm scanner, or if the scan is a fast helical scan (e.g. a one-beat helical scan), while the processing system may determine that a breath hold is required if the scan is a step and shoot scan. Other scan data may also be used to determine whether a breath hold is required; for instance, a breath hold may not be required if the scan data indicates that the subject will be sedated during the scan.

The camera 130 is configured to acquire video data 135 of a room in which the cardiac CT imaging procedure is being conducted. The video data 135 may be acquired during set-up of the room for the cardiac CT imaging procedure and/or during the cardiac CT imaging procedure. The video data may capture at least the CT scanner 120 (from a viewpoint from which the table of the CT scanner is visible). Preferably, the video data also captures areas of the room around the CT scanner 120. In some examples, the video data captures the entire room (e.g. using more than one camera if required).

The processing system 110 is configured to receive the video data 135. In Fig. 1, the video data is received directly from the camera 130; however, as the skilled person will readily appreciate, the video data may be received via another source storing the video data (e.g. a memory unit or a cloud server). In some examples, the processing system may receive and process the video data in real-time or near real-time (i.e. the processing system may receive and process each frame of the video data immediately after the frame is acquired by the camera). In some examples, the video data 135 may also form part of the scan data 115 used to determine the one or more criteria to be met.

Having determined the one or more criteria to be met and received the video data 135, the processing system 110 is configured to process the video data to determine whether the one or more criteria are met. The processing system is then configured to generate guidance based on the determination as to whether the one or more criteria are met.

As the skilled person will appreciate, the determination of whether the one or more criteria are met and the generation of the guidance will depend on the particular one or more criteria determined by the processing system 110.

For instance, if the one or more criteria comprise one or more criteria relating to a heart rate of the subject, the processing system 110 may be configured to process the video data 135 to estimate a heart rate and/or heart rate variability of the subject, and/or to detect any arrythmias (depending on the particular one or more criteria relating to the heart rate determined by the processing system). In some examples, the system may further comprise an electrocardiography (ECG) device configured to acquire an ECG signal for the subject, and the processing system may additionally or alternatively estimate the heart rate and/or heart rate variability, and/or detect any arrythmias by receiving and processing the ECG signal once this is available; however, in some cases, the processing system may be provide guidance before the ECG device has been connected.

In response to a determination that any criteria relating to the heart rate of the subject is not met (e.g. if the estimated heart rate falls outside the desired heart rate range, the estimated heart rate variability falls outside the desired heart rate variability range, and/or if any arrythmias are detected), the processing system 110 may be configured to generate guidance recommending an adjustment to the scan protocol (e.g. a switch from a step and shoot scan to a helical scan, with or without tube current modulation, as appropriate, or a reduction to the helical pitch).

If the one or more criteria comprises one or more criteria relating to use of an electrocardiography (ECG) device, the processing system 110 may be configured to process the video data 135 using an object recognition algorithm to identify any elements of an ECG device captured in the video data (e.g. electrodes, connection cables, etc.). If the one or more criteria comprises a criterion requiring the use of an electrocardiography device, the processing system may generate guidance recommending that an ECG is connected to the CT scanner 120 in response to failing to identify an ECG device in the video data, or in response to identifying that an ECG device captured in the video data is not connected to the CT scanner. If the one or more criteria comprises a desired position for each of a plurality of electrodes of an ECG device, the processing system may generate guidance indicating an adjustment to be made to the position of one or more electrodes in response to identifying that any electrode is not in a desired position with respect to the subject.

If the one or more criteria comprises one or more criteria relating to administration of a contrast agent, the processing system 110 may process the video data 135 using an object recognition algorithm to identify any elements of a contrast agent injection system (e.g. a pump, a needle, etc.) captured in the video data. If the one or more criteria comprises a criterion that a contrast agent is administered, the processing system may generate guidance recommending the administration of a contrast agent in response to failing to identify any elements of a contrast agent injection system. If the one or more criteria comprises a type of needle to be used for contrast agent administration, the processing system may generate guidance indicating the type of needle to be used in response to failing to identify the relevant type of needle in the video data.

If the one or more criteria comprises an amount of contrast agent to be administered, the processing system may be configured to process the video data to determine an amount of contrast agent in a contrast agent pump captured in the video data; in response to the amount of contrast agent to be administered exceeding the determined amount of contrast agent in the contrast agent pump (before administration of the contrast agent), the processing system may generate guidance recommending an amount of contrast agent to be added to the contrast agent pump. In some examples, if the one or more criteria comprises a contrast agent injection protocol that specifies the use of a saline chaser bolus and a desired amount of saline chaser, the processing system may also be configured to determine an amount of saline chaser in a secondary pump of the contrast agent injection system; in response to a determination that the amount of saline chaser is insufficient (i.e. less than the determined desired amount), the processing system may generate guidance recommending an amount of saline chaser to be added to the secondary pump.

In some examples, video data may be acquired during administration of the contrast agent, and the processing system may determine an amount of contrast agent actually administered by processing the video data to determine a change in the amount of contrast agent in the contrast agent pump after administration of the contrast agent. In response to a determination that the amount of contrast agent actually administered is less than the determined amount of contrast agent to be administered, the processing system may generate guidance indicating how much more contrast agent should be administered in order to achieve the determined amount.

The processing system may additionally determine whether a criterion defining a contrast agent injection protocol is met based on data received from the contrast agent pump (providing that the contrast agent pump is connected to the CT scanner 120).

If the one or more criteria comprises a criterion that a breath hold is required during the cardiac CT imaging procedure, the processing system 110 may be configured to process the video data 135 to analyze a breathing ability of the subject to determine a likelihood that the subject is able to complete a breath hold; in response to a determination that the subject is unlikely to be able to complete a breath hold, the processing system may generate guidance recommending that the subject performs additional breathing exercises, or suggesting an adjustment to the scan protocol to increase a redundancy in the CT imaging data acquired by the CT scanner 120 during the cardiac CT imaging procedure.

In some examples, the generated guidance may be further based on identifying any difference between a current position of the subject and a desired position of the subject. The processing system 110 may be configured to generate guidance based on any identified difference between the current position and the desired position by processing the video data 135 to identify the current position of the subject. Additionally or alternatively, the processing system may identify the current position of the subject by receiving and processing CT imaging data acquired by the CT scanner 120 during a localizer scan of the cardiac CT imaging procedure.

The desired position of the subject for a cardiac CT imaging procedure is a position in which the heart of the subject is at a center of rotation for the CT scanner 120. For instance, the identified difference between the current position and the desired position may be an estimated displacement between the center of rotation and a position of the heart of the subject in the current position. The desired position of the subject may be determined based on the type of scan; for instance, a TAVI scan requires that the aorta, as well as the heart, is close to the center of rotation.

The processing system 110 may then be configured to generate guidance indicating an amount and direction of movement of the subject required to achieve the desired position. In some examples, in response to identifying a difference between the current position of the subject and the desired position, the processing system may additionally or alternatively generate guidance indicating a change in position of the field of view of the CT scanner 120 that would result in the subject having the desired position relative to the field of view.

In some examples, the generated guidance may be further based on a determined amount of metal within a scan region of the subject (i.e. a region to be scanned during the main scan of the CT cardiac imaging procedure). The processing system 110 may be configured to generate guidance based on an amount of metal within the scan region by processing the video data 135 to determine an amount of metal within the scan region of the subject (e.g. by processing the video data using an object recognition algorithm to identify, within the scan region, any objects that are likely to be metal, e.g. cables, jewelry, etc.). Additionally or alternatively, the processing system may determine an amount of metal within the scan region by receiving CT imaging data acquired by the CT scanner 120 during a localizer scan of the cardiac CT imaging procedure, and processing the CT imaging data to identify any metal objects in the scan region. In response to identifying any metal objects in the scan region and outside of the subject's body (other than ECG electrodes and cables where an ECG device is used), the processing system may generate guidance recommending the removal of the identified metal object(s). In response to identifying any metal objects in the scan region and inside the subject's body, the processing system may generate guidance recommending an adjustment to the scan protocol (unless the scan protocol is already suitable); for instance, an adjustment to the tube voltage (kV) or a recommendation to use a spectral scanner.

In some examples, the processing system 110 may be further configured to process the scan data 115 to identify any inconsistencies in the scan data, and to generate guidance based on any identified inconsistencies. For instance, the processing system may be configured to process the scan data to determine a measure of the size or weight of the subject, and to determine whether an x-ray tube current (mA), voltage (kVp) and/or focal spot size of the CT scanner, as defined in the scan data, is suitable for the determined measure of the size/weight of the subject; in response to a determination that the x-ray tube current, voltage and/or focal spot size is not suitable, the processing system may generate guidance recommending an adjustment to the x-ray tube current, voltage and/or focal spot size, as appropriate.

The processing system 110 may also be configured to identify inconsistencies between the video data 135 and the scan data 115 and/or the determined one or more criteria. For instance, the processing system may be configured to process the video data to estimate a height, weight and/or gender of the subject; if any of these do not match corresponding information in the scan data 115, the processing system may generate guidance alerting an operator to a risk that the scan data is incorrect for the subject in the room (for instance, the subject in the room may not be the subject identified in the scan data, or one or more characteristics of the subject may have changed since the relevant portion of the scan data was acquired, and the scan protocol may need adjusting accordingly). In another example, if the one or more criteria comprises a criterion that a breath hold is required, but the video data indicates that the subject is sedated, the processing system may remove the criterion specifying that a breath hold is required, and generate guidance indicating that a breath hold is no longer required.

In some examples, in response to a determination that all criteria have been met (i.e. that no recommended adjustments have been identified), the processing system 110 may be configured to generate guidance advising the operator to proceed with the cardiac CT imaging procedure (e.g. advising the operator to begin a localizer scan if this has not yet been performed, or if adjustments made to the set-up require a further localizer scan to be performed, or advising the operator to proceed with the main scan).

Having generated the guidance, the processing system 110 is configured to control the feedback unit 140 to provide a user-perceptible output indicative of the generated guidance. Any suitable user-perceptible output for providing the generated guidance may be used (e.g. one or more of: an image, a light, a textual display, a sound and/or a vibration); as the skilled person will appreciate, the type of user-perceptible output will depend on a type of feedback unit 140 provided in the cardiac CT imaging system 100.

In Fig. 1, the feedback unit 140 is a display device, which may be configured to provide a user-perceptible output indicative of the generated guidance in the form of an image and/or a textual display (e.g. providing a written instruction or a checklist). In some examples, the generated guidance may be provided by controlling the display device to display the video data 135 (e.g. as a live video feed of the room) and an overlay indicating an area of the room for which an adjustment is recommended. In Fig. 1, the display device is a standalone monitor; however, the display device may alternatively be a display device of a scanner console for the CT scanner 120.

In some examples, the feedback unit 140 may additionally or alternatively comprise one or more lighting devices, and the processing system 110 may control at least one of the one or more lighting devices to illuminate an area of the room for which an adjustment is recommended in the generated guidance.

In some examples, the feedback unit 140 may additionally or alternatively comprise a speaker, and the processing system 110 may control the speaker to provide a sound (e.g. a spoken instruction) indicative of the generated guidance.

In some examples, the feedback unit 140 may comprise a plurality of feedback devices, each configured to provide a user-perceptible output. For instance, the feedback unit may comprise a first device, configured to provide a user-perceptible output alerting an operator to the fact that guidance has been generated, and a second device, configured to provide a user-perceptible output providing details of the guidance. The first device may be configured to provide a user-perceptible output that is likely to be perceived by the operator regardless of the position of the operator, such as a light illuminating an area of the room, or a wearable device (e.g. a wristband) configured to provide a sound and/or a vibration. In this way, the operator may be directed towards the second device to obtain details of the guidance, enabling the operator to be provided with the guidance quickly even when the operator is not initially paying attention to the second device.

In some examples, the processing system 110 may continue to receive and process the video data 135 after a user-perceptible output recommending one or more adjustments has been provided, in order to determine whether the recommended adjustment(s) has been made and whether any further adjustments are required. The processing system may repeat the steps of processing the video data, generating guidance and controlling the feedback unit 140 to provide a user-perceptible output indicative of the guidance until the generated guidance is to advise the operator to proceed with the cardiac CT imaging procedure.

In some examples, the processing system 110 may continue to receive and process the video data 135 even after the operator has been advised to proceed with the cardiac CT imaging procedure, in order to provide further guidance as appropriate (for instance, in case of movement of the subject during the cardiac CT imaging procedure).

In some examples, the processing system 110 may be configured to repeat the step of determining one or more criteria to be met in response to receiving any further scan data 115. For instance, if the processing system 110 initially determines one or more criteria to be met before a localizer scan of the subject has been performed, the processing system may repeat the step of determining one or more criteria to be met after a localizer scan has been performed, based on the CT imaging data acquired during the localizer scan. In response to determining one or more new criteria, the processing system may be configured to process the video data to determine whether the one or more new criteria are met, generating guidance and controlling the feedback unit as appropriate.

In some examples, the processing system 110 may be further configured to receive CT imaging data 125 for the subject acquired by the CT scanner 120 during the cardiac CT imaging procedure, and to process the CT imaging data to determine at least one measure of image quality.

The at least one measure of image quality may comprise one or more of: a measure of image sharpness, a measure of image contrast, a contrast-to-noise ratio, a signal-to-noise ratio, and/or a measure of visibility of an anatomical target of the cardiac CT imaging procedure. Further suitable measures of image quality will be apparent to the skilled person.

The processing system may be configured to control the feedback unit 140 to provide a user-perceptible output indicative of the at least one measure of image quality. This allows the operator to decide whether any aspects of the cardiac CT imaging procedure should be repeated. The processing system may be configured to receive and process the CT imaging data 125 immediately after the CT imaging data is acquired by the CT scanner 120, so that the subject is still in place on the table of the CT scanner when the user-perceptible output indicative of the at least one measure of image quality is provided.

Additionally or alternatively, the processing system may be configured to process the video data (and/or one or more of: CT imaging data acquired during a localizer scan of the cardiac CT imaging procedure, the scan protocol and/or subject data) and the at least one measure of image quality to identify any source of error (or to provide this data to another processing system for identifying any source of error). For instance, the video data and the at least one measure of image quality may be used as training data for a machine learning algorithm (e.g. a neural network), in order to train the machine learning algorithm to identify further sources of error. For instance, a machine learning model may be trained to predict CT imaging quality based on an input of video data for the imaging procedure (and/or one or more of: CT imaging data acquired during a localizer scan of the imaging procedure, the scan protocol and/or subject data). Video data for which the machine learning model predicts low CT imaging quality may be compared with video datala CT scan protocol for one or more imaging procedure that produced good quality CT imaging data to identify potential sources of error. Each imaging procedure for which video data is acquired and at least one measure of image quality is determined, as described above, may be used as training data to first train the model, or, for imaging procedures that occur after the model has been trained, as additional training data to update the model.

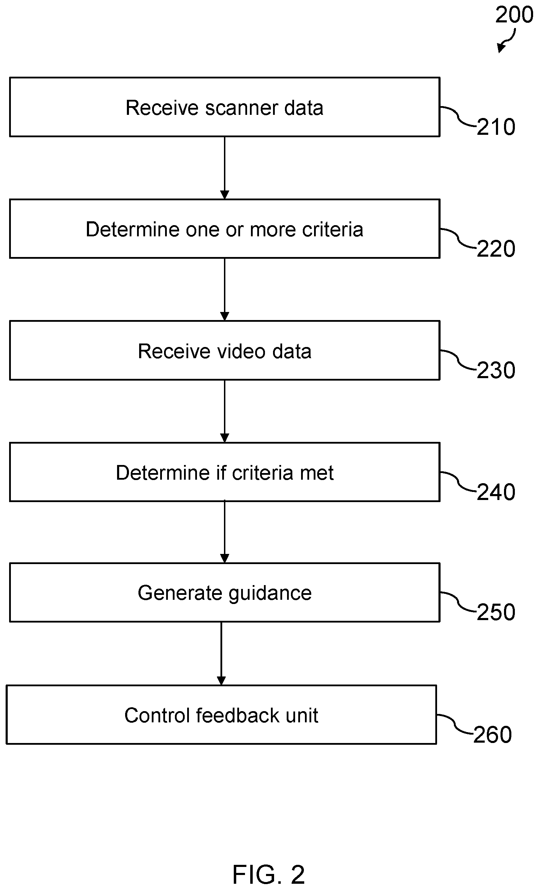

Fig. 2 illustrates a computer-implemented method 200 for providing guidance to an operator conducting a cardiac CT imaging procedure, according to an embodiment of the invention.

The computer-implemented method begins at step 210, at which scan data for the cardiac CT imaging procedure is received.

At step 220, the scan data is processed to determine one or more criteria to be met during the cardiac CT imaging procedure. The one or more criteria may comprise any of the criteria described above. In particular, the one or more criteria comprise at least one of: one or more criteria relating to a heart rate of a subject of the cardiac CT imaging procedure; one or more criteria relating to use of an electrocardiography device; and/or one or more criteria relating to administration of a contrast agent.

At step 230, video data of a room in which the cardiac CT imaging procedure is being conducted is received.

At step 240, the video data is processed to determine whether the one or more criteria are met.

At step 250, guidance is generated based on the determination as to whether the one or more criteria are met. In some examples, the guidance may be further based on any difference between a current position of the subject and a desired position, and/or on a determined amount of metal in a scan region of the subject, as described above.

At step 260, a feedback unit is controlled to provide a user-perceptible output indicative of the generated guidance. In some examples, the feedback unit comprises one or more lighting devices, and step 260 comprises controlling at least one of the one or more lighting devices to illuminate an area of the room for which an adjustment is recommended. In some examples, the feedback unit comprises a display device, and step 260 comprises controlling the display device to display the video data and an overlay indicating an area of the room for which an adjustment is recommended.

In some examples, the computer-implemented method 200 further comprises a step (not shown in Fig. 2) of acquiring CT imaging data for the subject; and processing the CT imaging data to determine at least one measure of image quality.

In some examples, the computer-implemented method 200 may further comprise a step of controlling the feedback unit to provide a user-perceptible output indicative of the at least one measure of image quality.

In some examples, the computer-implemented method 200 may further comprise a step of processing the video data and the at least one measure of image quality to identify any source of error.

It will be understood that the disclosed methods are computer-implemented methods. As such, there is also proposed a concept of a computer program comprising code means for implementing any described method when said program is run on a processing system.

The skilled person would be readily capable of developing a processing system for carrying out any herein described method. Thus, each step of a flow chart may represent a different action performed by a processing system, and may be performed by a respective module of the processing system.

As discussed above, the system makes use of a processing system to perform the data processing. The processing system can be implemented in numerous ways, with software and/or hardware, to perform the various functions required. The processing system typically employs one or more microprocessors that may be programmed using software (e.g. microcode) to perform the required functions. The processing system may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processing system may be associated with one or more storage media such as volatile and non-volatile computer memory such as RAM, PROM, EPROM, and EEPROM. The storage media may be encoded with one or more programs that, when executed on one or more processing systems and/or controllers, perform the required functions. Various storage media may be fixed within a processing system or controller may be transportable, such that the one or more programs stored thereon can be loaded into a processing system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processing system may be implemented by a single processing system or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A computer-implemented method (200) for providing guidance to an operator conducting a cardiac CT imaging procedure, the computer-implemented method comprising:
receiving scan data (115) for the cardiac CT imaging procedure;
processing the scan data to determine one or more criteria to be met during the cardiac CT imaging procedure, the one or more criteria comprising at least one of:
one or more criteria relating to a heart rate of a subject of the cardiac CT imaging procedure;
one or more criteria relating to use of an electrocardiography device; and/or
one or more criteria relating to administration of a contrast agent;
receiving video data (135) of a room in which the cardiac CT imaging procedure is being conducted;
processing the video data to determine whether the one or more criteria are met;
generating guidance based on the determination as to whether the one or more criteria are met; and
controlling a feedback unit (140) to provide a user-perceptible output indicative of the generated guidance.

2. The computer-implemented method (200) of claim 1, wherein the one or more criteria further comprises at least one criterion relating to a breathing state of the subject.

3. The computer-implemented method (200) of claim 1 or 2, wherein:
the computer-implemented method further comprises processing the video data (135) and/or CT imaging data acquired during a localizer scan of the cardiac CT imaging procedure to identify any difference between a current position of the subject and a desired position; and
the generated guidance is further based on any identified difference between the current position and the desired position.

4. The computer-implemented method (200) of any of claims 1 to 3, wherein:
the computer-implemented method further comprises processing the video data (135) and/or CT imaging data acquired during a localizer scan of the cardiac CT imaging procedure to determine an amount of metal within a scan region of the subject; and
the generated guidance is further based on the determined amount of metal.

5. The computer-implemented method (200) of any of claims 1 to 4, wherein:
the feedback unit (140) comprises one or more lighting devices; and
the step of controlling the feedback unit to provide a user-perceptible output indicative of the generated guidance comprises controlling the one or more lighting devices to illuminate an area of the room for which an adjustment is recommended.

6. The computer-implemented method (200) of any of claims 1 to 5, wherein:
the feedback unit (140) comprises a display device; and
the step of controlling the feedback unit to provide a user-perceptible output indicative of the generated guidance comprises controlling the display device to display the video data and an overlay indicating an area of the room for which an adjustment is recommended.

7. The computer-implemented method (200) of any of claims 1 to 6, further comprising:
acquiring CT imaging data (125) for the subject; and
processing the CT imaging data to determine at least one measure of image quality.

8. The computer-implemented method (200) of claim 7, further comprising controlling the feedback unit (140) to provide a user-perceptible output indicative of the at least one measure of image quality.

9. The computer-implemented method (200) of claim 7 or 8, further comprising processing the video data (135) and the at least one measure of image quality to identify any source of error.

10. The computer-implemented method (200) of any of claims 1 to 9, wherein the one or more criteria relating to the heart rate of the subject comprises one or more of:
a desired heart rate range;
a desired heart rate variability range; and/or
a criterion that no arrythmias occur.

11. The computer-implemented method (200) of any of claims 1 to 9, wherein the one or more criteria relating to the use of an electrocardiography device comprises one or more of:
a criterion defining whether or not an electrocardiography device should be used; and/or
a desired position for each of a plurality of electrodes of an electrocardiography device.

12. The computer-implemented method (200) of any of claims 1 to 11, wherein the one or more criteria relating to administration of a contrast agent comprises one or more of:
a criterion defining whether or not a contrast agent is to be administered;
an amount of contrast agent to be administered;
a criterion defining a contrast agent injection protocol; and/or
a type of needle to be used for contrast agent administration.

13. A computer program product comprising computer program code means which, when executed on a computing device having a processing system, cause the processing system to perform all of the steps of the method (200) according to any one of claims 1 to 12.

14. A processing system (110) for providing guidance to an operator conducting a cardiac CT imaging procedure, the processing system being configured to:
receive scan data (115) for the cardiac CT imaging procedure;
process the scan data to determine one or more criteria to be met during the cardiac CT imaging procedure, the one or more criteria comprising at least one of:
one or more criteria relating to a heart rate of a subject of the cardiac CT imaging procedure;
one or more criteria relating to use of an electrocardiography device; and/or
one or more criteria relating to administration of a contrast agent;
receive video data (135) of a room in which the cardiac CT imaging procedure is being conducted;
process the video data to determine whether the one or more criteria are met;
generate guidance based on the determination as to whether the one or more criteria are met; and
control a feedback unit (140) to provide a user-perceptible output indicative of the generated guidance.

15. A cardiac CT imaging system (100), comprising:
the processing system (110) of claim 14;
a CT scanner (120);
a camera (130) configured to acquire video data of a room in which a cardiac CT imaging procedure is being conducted; and
a feedback unit (140) configured to provide a user-perceptible output.
